# EUROPEAN PATENT APPLICATION

(11) **EP 1 160 332 A1**
(43) Date of publication of application: **05.12.2001**
(21) Application number: 00201923.0
(22) Date of filing: 30.05.2000
(51) Int. Cl.: C12Q 1/68, C12Q 1/37, C12Q 1/25

(54) **Methods for detecting enzymatic activity or detecting resistance to enzyme inhibitors**

(71) Applicant: Amsterdam Support Diagnostics B.V., 1105 BA Amsterdam (NL)
(72) Inventor: Kim, Baek, Rochester, NY 14168 (US); Baan, Elly, 1104 GL Amsterdam (NL); de Ronde, Anthonij, 1107 DE Amsterdam (NL); Goudsmit, Jaap, 1075 CX Amsterdam (NL); van Gemen, Bob, 1326 HV Almere (NL); Patel, Daksha, Victor NY 14564 (US)
(74) Representative: Prins, Adrianus Willem

(57) **Abstract**

The invention relates to the detection or diagnosis of genetically based enzymatic activity and/or resistance to enzyme inhibitors. The invention provides a method using an enzyme-based assay which is faster, less expensive and less facility-dependent than currently available methods for measuring the resistance to enzyme inhibitors are. The invention provides for testing the inhibiting activity of a compound on an enzyme, or the resistance of said enzyme against said inhibiting activity.

## Description

The invention relates to the detection or diagnosis of genetically based enzymatic activity and/or resistance to enzyme inhibitors.

Enzymes play essential roles in numerous biological processes. Substrate specificity, or the ability to discriminate among many potential substrates is central to the function of enzymes. Knowing an enzyme's substrate specificity gives valuable insights into its biological function but also provides the basis for potent substrate and inhibitor design and perform resistance studies relating to those inhibitors. Many enzymes are already known in the field of biology but many more will become known in the near future, enhancing all these studies as well. For example, the multitude of genome sequencing projects currently in progress will ensure the discovery for many new enzymes in the coming decades. A wealth of uncharacterised open reading frames, several thousands from bacterial or fungal species and up to a hundred thousand or more for plant or animal genomes, will be available soon. Many of these open reading frames will be found to encode enzymes or proteins with catalytic function.

Since enzymes play such a central role, drugs affecting or interfering with their action, commonly known as inhibitors, agonists or antagonists, are often central in modulating essentially biological processes, be it *in vivo* use in for example therapy or *in vitro* use in for example cell culture work, and many drugs or compounds are known and used that interfere with such processes by inhibition. Of course, nature--ever evolving―continues to develop enzymes resistant against such inhibitors and the present application deals among others with detecting such resistance's and the genetic bases thereof.

For example, current methods for the diagnosis of drug resistance to protease inhibitors heavily rely on genotype- and phenotype-based analyses. For example, the genotyping of human immunodificiency virus of samples obtained from patients employs analysis of the sequence encoding the specific viral protease. However, the sequence analysis data can not directly be related to a resistance profile of protease inhibitors for the virus. To obtain a resistance profile additional phenotypic analysis is required. The phenotype-based analysis employs *in vitro* tissue cultures of the viral samples obtained from the patient in the presence of various concentrations of viral protease inhibitors. This method is not only expensive to perform but also highly dependent on skills and facilities available to the researchers, due to the infectious nature of *in vitro* cultured HIV-1 virus. Although in essence non-culture methods are known (for example WO99/66068), these still rely on whole virus to begin with. In addition to these limitations, genotype- and phenotype based methods in general require a long period of time for their analyses. It takes long before a specific enzyme's resistance against a inhibitor is found, and for example the proper decision for treatment of patients with anti-viral drugs that a patient's virus enzymes are sensitive to, will be delayed until completion of these diagnostic analyses. Consequently, therapy of the patient with appropriate protease inhibitors has to be postponed accordingly, and during the delay the patient's virus can acquire additional resistance conferring mutations. The above described diagnostic practice shows the need for assays capable of assessing an enzyme's inhibitor resistance profile in a short period of time.

The invention provides a method using an enzyme-based assay which is faster, less expensive and less facility-dependent than currently available methods for measuring the resistance to enzyme inhibitors are. The invention provides for testing the inhibiting activity of a compound on an enzyme, or the resistance of said enzyme against said inhibiting activity.

In one embodiment, the invention provides a method for determining resistance of an enzyme against an inhibiting activity of a compound comprising amplification of a nucleic acid encoding said enzyme or at least one functional fragment thereof further comprising *in vitro* translation of said amplified nucleic acid to generate said enzyme or fragment, further comprising contacting said *in vitro* translated enzyme or fragment with a substrate for said enzyme further comprising contacting said enzyme or fragment with at least one compound and measuring the inhibiting activity of said compound on said enzyme or fragment by measuring enzymatic activity directed against said substrate. Herein a method is provided wherein enzymes can be tested for their resistance against inhibiting compounds, and, for example when it has been determined that said enzyme is resistant against a distinct compound, it is also provided to test whether said enzyme may or may not be inhibited by other compounds. Thus, in another embodiment the invention provides a method for determining an inhibiting activity of a compound towards an enzyme comprising amplification of a nucleic acid encoding said enzyme or at least one functional fragment thereof further comprising *in vitro* translation of amplified nucleic acid, contacting translated enzyme or fragment with a substrate and said compound and measuring the inhibiting activity of said compound by measuring enzymatic activity. Of course, a method as provided by the invention can be used in screening for example a library of test enzymes for their likelihood of being inhibited by a test compound or compounds, and can thus be used for identifying an enzyme inhibitable by such a compound and leads thus to an enzyme identifiable by a use according to the invention. Likewise, the invention provides use of a method according to the invention in screening a library of test compounds for their likelihood of having inhibiting activity towards an enzyme and provides such use for identifying a compound having inhibiting activity towards an enzyme, and leads thus to a compound identifiable by a use according to the invention. Of course, such compounds are used in preparing a pharmaceutical composition. A pharmaceutical composition comprising a compound identified according to the invention and a method of treatment of a patient is thus also provided, e.g. useful in HIV patients harbouring viruses resistant to treatment with other drugs.

Methods or assays are provided herein that are in essence applicable to any enzyme, in particular to any protease, either virus, micro-organism, plant, animal or human encoded. In a preferred embodiment a method is provided that comprises an functional protease (PR) or reverse transcriptase (RT) activity based assay to measure drug resistance of said protease to protease inhibitors, or of said transcriptase to transcriptase inhibitors like those used in the therapy of HIV. Of course, when herein a method to measure inhibiting activity of a compound or to measure resistance of an enzyme against the inhibiting activity of a compound is discussed, analogous methods, wherein the phrase "inhibitor" can be replaced by "agonist" or "antagonist", and inhibiting activity likewise, are also meant, as the person skilled in the art shall appreciate.

The invention provides a method to measure activity of an enzyme or to measure resistance of an enzyme against the inhibiting activity of a compound, preferably a drug or an enzyme inhibitor, preferably a protease inhibitor, comprising amplification of a nucleic acid encoding said enzyme or at least a functional (e.g. catalytic) fragment thereof by transcription further comprising *in vitro* translation of said amplified nucleic acid or amplicon to generate said enzyme or said functional fragment thereof, further comprising contacting said enzyme or functional fragment thereof with a suitable substrate for said enzyme, and measuring enzymatic activity on said substrate, and, in those cases where resistance is measured, further comprising contacting said enzyme or functional fragment thereof with at least one compound and measuring the inhibiting activity of said compound on said enzyme or functional fragment thereof by measuring enzymatic activity on said substrate. *In vitro* translation for examples comprises submitting a nucleic acid encoding the enzyme or fragment under study to an essentially cell free translation system, such as a (rabbit) reticulocyte lysate, wheat germ lysate, E. coli lysate or other eukaryotic or prokaryotic *in vitro* translation system whereby the desired (poly)peptide with enzymatic activity is produced, i.e. translated from its corresponding open reading frame or frames. Said (poly)peptide is contacted with its substrate, in the absence or presence of a possibly inhibiting compound or drug or inhibitor, e.g. by mixing the (poly)peptide sample with substrate (and compound), or by providing the substrate, and/or compound and/or enzyme or fragment thereof on a solid support that is than contacted with any of the other components in dilution, or by any other suitable method where compound is added to the *in vitro* translation systen, and enzymatic activity is measured. Of course, necessary control samples are included.

In a preferred embodiment, the invention provides a method comprising *in vitro* amplification for example by an amplification method such as (reverse transcriptase)-polymerase chain reaction ((RT)-PCR), nucleic acid sequence based amplification (NASBA), strand displacement amplification (SDA), transcription mediated amplification (TMA) of a nucleic acid encoding said enzyme or at least a functional (e.g. catalytic) fragment thereof by *in vitro* transcription. Generally available in vitro transcription and translation systems are conveniently used to express enzyme or fragment thereof directly from the amplified DNA's or RNA's or amplicon, which enzyme or fragment can than immediately be tested or stored for further use. Of course, coupled *in vitro* transcription/translation systems, such as one-tube reactions using for example T7 RNA polymerase constructs, are preferred. An added advantage is that an *in vitro* transcribed and translated enzyme or fragment thereof can easily be handled, with minimal sample preparation in between the various steps of the process.

Which substrate should be used with which enzyme is generally well known in the art, since most enzymes are indeed specific for one or a few substrates or classes thereof. For protease's, in general (poly)peptides should be selected as substrate, whereby it is equally well suitable to use native proteins or peptides or synthetic versions thereof.

In a preferred embodiment, the *in vitro* translated peptide comprising the enzyme or fragment thereof under study functions as substrate as well. Sometimes protease's have self-splicing activity, which means that the *in vitro* produced enzyme or fragment thereof in itself can function as substrate in an assay as provided herein. Protease activity of another protease and the inhibiting activity of compounds thereon can also be measured by using as substrate a (synthetic) (poly)peptide provided with a marker or reporter group, such as GFP or another functional peptide, or AMC that fluoresces when a peptide is cleaved off, an antigen that allows immunological detection, a His tag or any other reporter group or tag that allows easy detection of proteolytic cleavage or other related enzymatic activity. Of course, one can also test combinatorial (poly)peptide libraries, the various peptides contained in the library each acting as substrate.

For distinct enzymes distinct substrates are often suitable. Substrates such as peptides relate to enzymes such as protease's (but also to enzymes that are for example capable of post-translational modifications), carbohydrates relate to for example amylases or glycosyl transferase, lipids to lipases, nucleic acids to transcriptases, polymerases, replicases or restriction enzymes, and so on, whereby the specific enzyme under study dictates the choice of substrate to be used. Again, substrates can be provided with reporter groups well known in the art, and again, substrate libraries, such as nucleic acid libraries can be applied. The invention for example provides for testing the inhibiting activity of a nucleic acid or other compound on a polymerase or restriction enzyme, or the resistance of said enzyme against said inhibiting activity.

In a further embodiment the invention provides a methods as employed in the detailed description herein to measure resistance of protease activity to protease (PR) inhibitors. Of course, after modifications to serve the specific needs of the enzyme under study, the method as provide here essentially applies to reverse transcriptase (RT) and viral integrase as well, as well as to other viral or non-viral enzymes to be tested. In a translation based PR resistance assay (TPRA) the PR gene and its flanking regions are directly amplified from the samples obtained from the patient. This amplification uses for example a 5' primer containing specific sequences that allow in vitro transcription and translation of (viral) enzymes, i.e. a promotor sequence of RNA polymerase like T7 or SP6, a translation facilitating sequence like a Kozak or Shine-Delgarno sequence, an ATG-start codon and a sequence matching the sequence of the region to be amplified; a 3' primer with or without a module for detection of the produced PR protein, i.e. a sequence matching the region to be amplified followed by a stop-codon or followed by a region encoding the detection module and a stop-codon.

Commercially available in vitro transcription and translation systems are used to express functional enzymes directly from the amplified DNA's or RNA's. Also in case of amplification and transcription/translation of the HIV-1 protease the whole region of PR and RT, or part thereof, can be amplified and translated, if so desired. The cleavage site between PR and RT in PR-RT polyprotein is processed by self-proteolytic activity of the protease in the expressed PR-RT-proteins and may be aided by the intrinsic proteolytic activity of an *in vitro* expression system like the E. coli system. The produced proteins and their cleavage products can be visualised by for example radioactive labelling of the protein, by probes that detect specific amino acid sequences of the expressed PR proteins and/or cleavage products. The HIV-1 PR-RT proteins can *in vitro* be translated and incubated in the absence or presence of increasing concentrations of protease inhibitors. In this reaction, the self-processing of the PR-RT protein containing wild type PR is inhibited by anti-PR drugs, whereas the self-processing of the PR-RT proteins containing drug resistant PR sequences is not inhibited by PR inhibitors. The level of the remaining unprocessed PR-RT proteins as well as the levels of the processed products that will be monitored during the course of the reaction will be a diagnostic indicator for the drug resistance of the HIV-1 viruses collected from the patients.

In another embodiment, the invention provides for example a PR resistance assay in intermolecular cleavage reaction with PR substrates. Herein, the protease encoding sequences are amplified in the same manner as described above. Also identical to the previous section is the translation of the amplified RNA or DNA into a functional protein or peptide that can be further investigated for its function in the presence or absence of protease inhibitor drugs. The expressed PR will be incubated with substrate molecules containing for example separation modules such as His tag and GST to allow bound/free separation, and/or PR cleavage sites like those of HIV-1 protease or thrombin or indicator or detection modules or reporter groups such as green fluorescent protein (GFP). The translated enzyme or functional fragment thereof will process the cleavage sites between separation and detection modules of the substrates, physically separating these two modules. Alternatively, peptide substrates can be used. The expressed viral PR will be incubated with substrate molecules containing a peptide of a sequence encompassing a cleavage site of the protease. Covalently linked to the peptide are a fluorophore and a quencher molecule, each on a different side of the cleavage site. Cleavage by protease of the peptide will physically separate fluorophore and quencher, resulting in the emission of fluorescent light after excitation.

Detection methods of inter-molecular processing of the substrates by PR can be achieved in the following ways:
The protein substrates incubated with in vitro synthesised PR in the absence or presence of increasing concentrations of PR inhibitors will be applied to Western Blotting analysis that detects cleavage of the substrate or to affinity column resins that allow separation of the unprocessed full length substrate molecules and processed smaller substrate molecules. The processed substrate molecules that would loose their separation modules will not be detected by Western analysis with probes for the separation modules but will be detected with probes for the detection modules. The unprocessed full length and processed molecules are monitored in the reactions performed in the presence or absence of protease inhibitors. The unprocessed full length molecules and processed molecules which both contain a detection module and of which only the unprocessed full length molecules contain the separation module can be identified by Western Blotting analysis according to their size difference. The reactions with wild type PR and PR inhibitors show higher levels of full length substrates than the reactions with drug resistant PR and PR inhibitors.

The processed substrate molecules that loose the separation modules will not bind to the resins that recognise the separation modules. Therefore, in the column separation, the unbound processed substrate will be detected in the flow-through (F-T) fractions, whereas the unprocessed substrate that contains the separation modules will bind to the resin. The level of substrates processed by PR will be determined by measuring the level of detection domain (i.e. GFP) in the flow-through fraction. In the reaction with wild type PR and PR inhibitor, no detection domain will be detected in the F-T fraction, whereas, in the reaction with drug resistant PR and PR inhibitor, high level of the detection domain will be detected in the F-T fraction.

Assessment of resistance to protease inhibitor of the sequence under investigation using peptide substrates can be performed in the following way. The peptide substrate will be incubated with in vitro synthesised PR in the absence or presence of increasing concentrations of PR inhibitors. The intensity of the fluorescence of the peptide substrate will be real time monitored by using a fluorimeter or by visualisation by UV-light. The extent of the increase in fluorescence intensity in the reaction mixture is proportional to the protease activity in the reaction.

Of course, the methods and embodiments herewith shown with protease inhibitors of the viral protease can equally well be applied with other enzyme/substrate combinations and possibly inhibiting compounds related thereto, and are suited for high-throughput screening. The invention is further explained in the detailed description without limiting the invention thereto.

### Detailed description.

### Summary of materials and ingredients used in the examples.

All basic ingredients for buffers (Tris, MgCl₂, KCl, etc.) were purchased from Merck Nederland BV, Postbox 8198, 1005 AD Amsterdam, The Netherlands or Sigma-Aldrich Chemie BV, Stationsplein, Postbox 27, 3330 AA Zwijndrecht, The Netherlands. Amplitaq Gold DNA polymerase was purchased from PE Applied Biosystems, Benelux, Hoogeveenenweg 100, Postbox 305, 2910 AH Nieuwerkerk a/d IJssel, The Netherlands. dNTP's and NTP's were purchased from Amersham Pharmacia Biotech, Benelux, PO Box 1386, 4700 BJ Roosendaal, the Netherlands. Ni-NTA His bind resin was purchased from NIH AIDS Research Reference Reagent Program, 621 Lofstrand Lan, Rockville, MD 20850, USA. HIV protease substrate was purchased from Molecular Probes, Poortgebouw, Rijnsburgerweg 10, 2333 AL Leiden, the Netherlands. Wheat Germ and Rabbit Reticulocyte transcription/translation systems were purchased from Promega Benelux BV, PO Box 391, 2300 AJ Leiden, The Netherlands and E.coli transcription/translation systems were purchased from Ambion, via Sanbio BV, PO Box 540, 5400 AM Uden, The Netherlands. Oligonucleotide primers were purchased at different oligonucleotide suppliers and were usually purified by the supplier and tested for functionality in a PCR reaction with a known amount of input.
Nucleic acid was isolated from (clinical) samples by a variety of methods. One of the methods that is very suitable for this purpose is the method described by Boom et al. (1990)¹.
¹ *J Clin Microbiol* 1990 Mar;28(3):495-503 Rapid and simple method for purification of nucleic acids. Boom R, Sol CJ, Salimans MM, Jansen CL, Wertheim-van Dillen PM, van der Noordaa J

The sequences of the oligonucleotide primers that are used in the examples are shown in table 1.

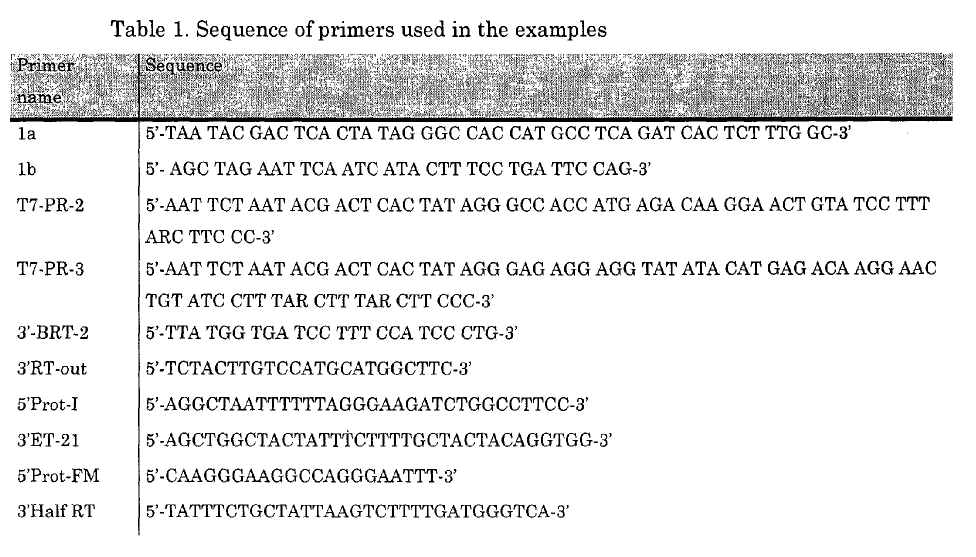

### Example 1:

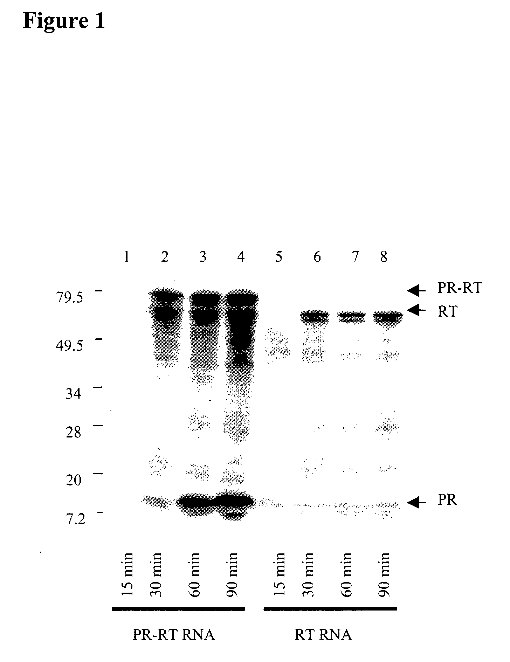

In this example we amplified the full length PR-RT sequence from a plasmid containing part of HIV 1 HXB2 sequence by using primer 1a for the 5' primer and primer 1b for the 3'primer, generating a 1.9 kB fragment. The amplified DNA's were directly applied to an in vitro transcription system (Promega, PCR product 1 µl, RNA Polymerase buffer 5 µl, rNTP [25mM each] 5 µl, T7 RNA Polymerase 5 µl, RNasin 2.5 µl, H₂O 31.5 µl, incubate at 37°C for 2hr), and the produced RNA's were identified by 0.9% agarose gel electrophoreses, followed by translation into proteins by rabbit reticulocytes in vitro translation reagents of the TnT Quick Coupled Trancription/Translation System of Promega with ³⁵S-methionine added. Samples of the translated products taken at different incubation times were analyzed by 12% SDS-polyacrylamide gel electrophoreses, and the dried gel was applied to X-film for autoradiography (see figure 1)

*Interpretation of figure 1:* The full length PR-RT fusion sequences are translated into protein in the rabbit reticulocyt system and subsequently processed, generating two cleavage products, PR and RT.

### Example 2:

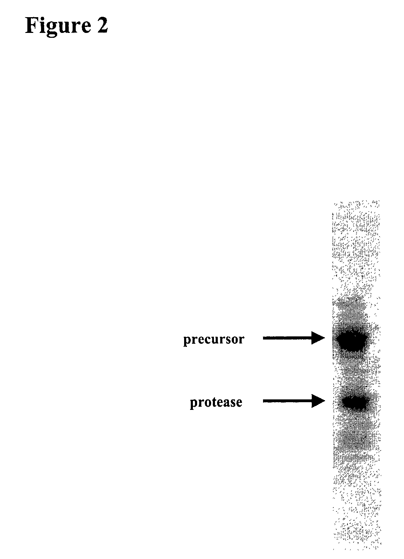

In this example we amplified part of the HIV-1 PR-RT sequence from a plasmid containing HIV 1 HXB2 sequence, using primer T7-PR-2 for the 5' primer and primer 3'-BRT-2 for the 3' primer generating a 0.7 kB fragment. For transcription and translation 500 ng of the amplified DNA was purified by phenol/chloroform extraction, ethanol precipitation and used as a template in a TnT T7 Coupled Wheat Germ Extract System, which was used according to the protocol of the manufacturer, with ³⁵S-methionine, added. The translated products were analyzed by 15% SDS-polyacrylamide gel electrophoreses, and the dried gel was applied to X-film for autoradiography (see figure 2).

*Interpretation of figure 2:* When the length of the amplified PR-RT fragment is reduced till 24 kD, the PR was still cleaved out of its precursor. The wheat germ transcription/ translation system is a suitable system to produce active protease.

### Example 3:

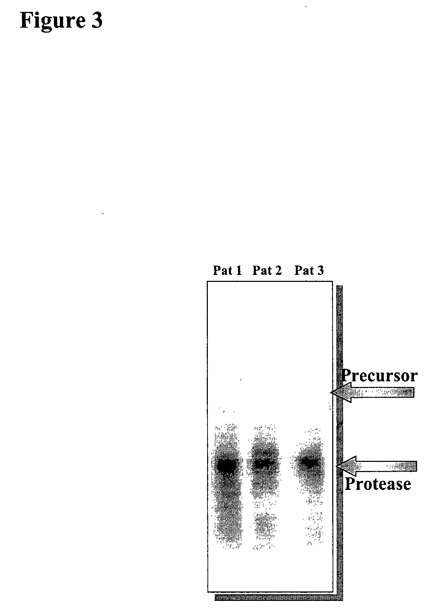

In this example we isolated HIV-1 RNA from HIV 1 positive patients with or without resistance conferring mutations for protease inhibitors like Saquinavir and Ritonavir according the Boom-protocol. The isolated RNA is transcribed into cDNA by MMLV-RT using primer 3'RT-out for 3' priming, followed by amplification using primer 5'Prot-1 for the 5'primer and primer 3'ET21 for the 3'primer. This PCR product is used in a second PCR using the primers 5'Prot-FM and 3'Half RT, followed by a third PCR creating the truncated PR-RT, using primers T7-PR-3 and 3'BRT-2, generating a 0.7 kB fragment. The amplified DNA was used as a template in PROTEINscript-PRO, an E.coli transcription/translation coupled system used according to the manufacturer's protocol, with ³⁵S-methionine added. The translated products were analyzed by 15% SDS-polyacrylamide gel electrophoreses, and the dried gel was applied to X-film for autoradiography (see figure 3).

*Interpretation of figure 3*: In the *E. coli* transcription/translation system the precursor protein is cleaved completely in the absence of protease inhibitors.

### Example 4:

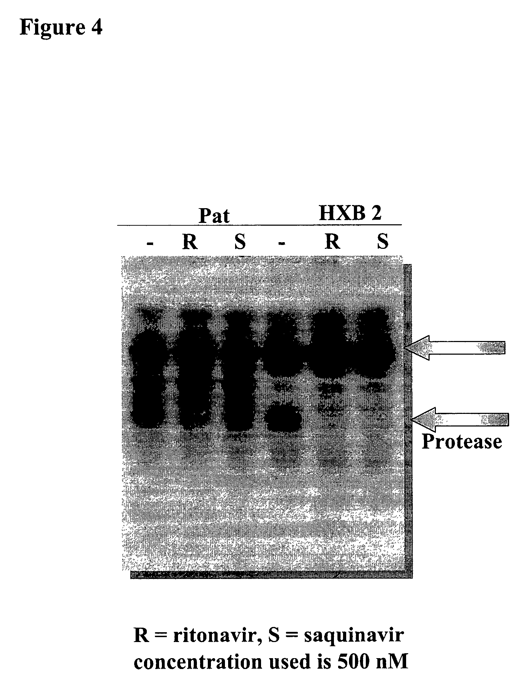

In this sample we prepared c-DNA from a HIV 1 positive patient with resistance conferring mutations against protease inhibitors as described under example 3 but in the third PCR we used primers T7-PR-2 and 3'BRT-2, generating a 0.7 kB fragment. The amplified DNA was used as a template in a TnT T7 Coupled Wheat Germ Extract System used according the protocol of the manufacturer with Ritonavir or Saquinavir and ³⁵S-methionine added. As a control we used the PCR product of example 2. The translated products were analyzed by 15% SDS-polyacrylamide gel electrophoreses, and the dried gel was applied to X-film for autoradiography (see figure 4).

*Interpretation figure* 4: Protease inhibitors inhibit cleavage of the PR-RT precursor when HIV-1 genes without resistance conferring mutations are used in the amplification. In case genes with resistance conferring mutations are used, the protease inhibitors are not able to inhibit the cleavage.

### Example 5:

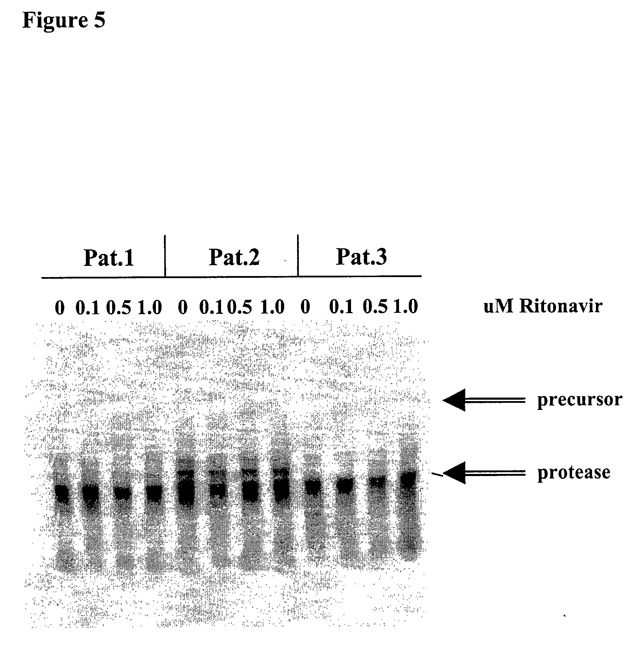

In this example we repeated the transcription/translation experiment of example 3, but now with increasing concentrations of Ritonavir. For the results see figure 5.

*Interpretation figure 5:* In spite of the presence of protease inhibitors all precursors, both the ones with resistance conferring mutations as well as the ones without, are cleaved completely. The scavenger protease in the E. coli extract, which cleaves the sequence X-Pro is responsible for the complete cleavage of the PR-RT cleavage site, and is not blocked by inhibitors, directed against HIV-1 protease.

### Example 6.

To test cleavage of a protein substrate containing a proteolytic cleavage site, thrombin and its cleavage site are used. The constructed protein substrate contains a separation module (6 histidines, His tag), thrombin cleavage site (CL) and a detection module (green fluorescent protein, GFP). The proteolytic processing of this protein substrate molecule by thrombin separates the detection module and the separation module. The thrombin substrate, His-CL-GFP, is incubated with thrombin, and two different methods are employed to detect the cleavage of this substrate molecule by thrombin: (A) Western analysis for separation module (B) Ni⁺⁺-chelated affinity column analysis. The cleavage reactions are also performed in the presence of a competitive peptide thrombin inhibitor.

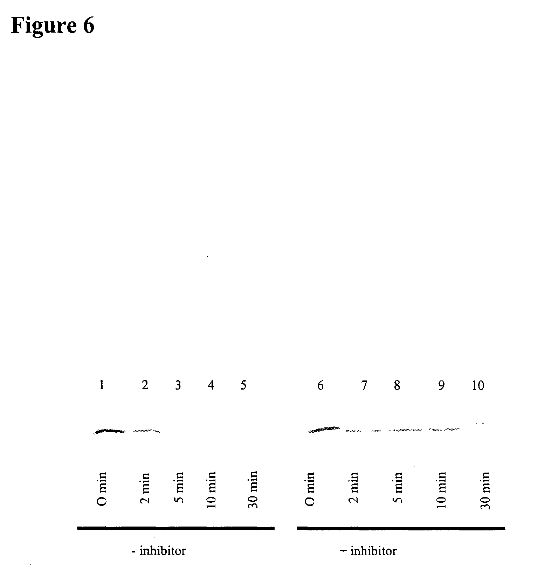

*Interpretation figure 6:* An anti-His tag probe conjugated with alkaline phosphatase (Qiagen) is used to monitor proteolytic processing of the protein substrate by thrombin. The processing of the protein substrate by thrombin causes loss of His tag, and the processed substrate can not detected by the probe. As seen in Figure 6 the level of the full length protein substrate gradually decreases during the incubation with thrombin, whereas the level of the full length protein substrate is sustained in the presence of thrombin inhibitor.

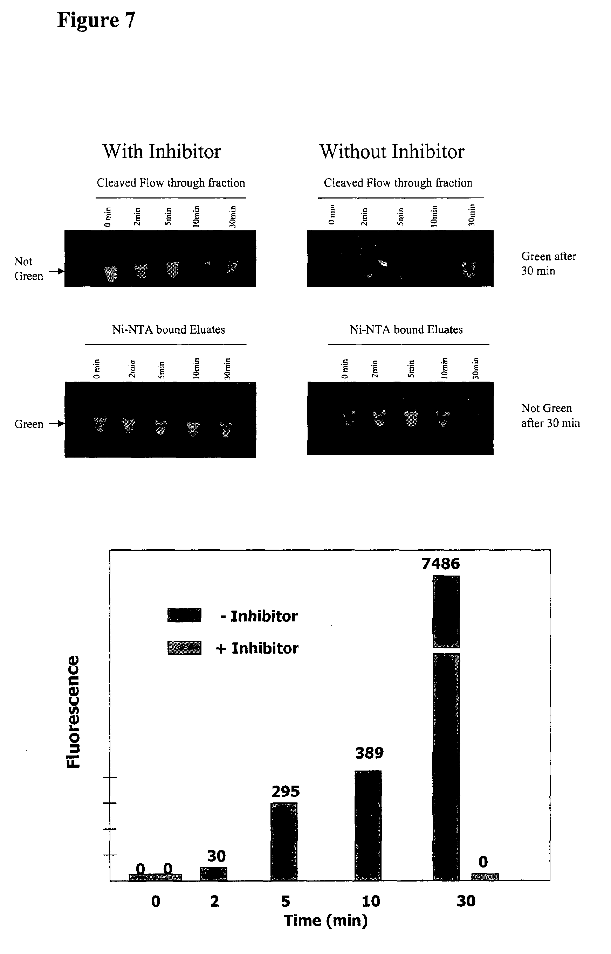

*Interpretation figure* 7: The Ni⁺⁺ - chelated resin is used to separated the unprocessed protein substrate that binds to the resin and the processed substrate that does not bind to the resin. The proteolytic reactions of the protein substrate by thrombin are applied to the Ni⁺⁺ - chelated column, and the flow-through (F-T) fractions that contain processed protein substrates are analyzed. The fluorescence of the processed substrate is monitored by 1) visualization with UV light (Figure 3B) and 2) fluorescence meter (Figure 3C). As seen in Figure 3(B) and 3(C), the level of the processed substrate in the F-T fraction increases during the incubation with thrombin. However, the level of processed substrate is sustained during the reaction in the presence of the inhibitor.

### Example 7:

A peptide substrate containing a PR cleavage sequence and covalently linked fluorophore and quencher molecules (on different sides of the cleavage site) was incubated with purified PR. The processing of this non-fluorescent peptide substrate will generate the cleaved peptide product that becomes fluorescent as shown by visualization with UV light (table 2)

**Table 2.**

| Fluorescence measured after incubation of purified protease with the peptide substrate described in example 7 | | |
|---|---|---|
| Reaction | Protease concentration (ng/assay) | Fluorescence |
| 1 | 0 | - |
| 2 | 1 | - |
| 3 | 3 | - |
| 4 | 10 | + |
| 5 | 30 | ++ |
| 6 | 90 | +++ |
| 7 | 270 | ++++ |
| 8 | Negative control | - |

*Interpretation of table 2:* In the absence of purified protease no fluorescence is observed, whereas in the presence of increasing amounts of protease the fluorescence becomes more intense.

### Legends to figures

Figure 1. Autoradiograph of the ³⁵S labeled in vitro transcription/translation products of HIV-1 PR-RT and HIV-1 RT sequences.

Figure 2. Autoradiograph of the ³⁵S labeled in vitro transcription/translation products of HIV-1 PR-RT sequence.

Figure 3. Autoradiograph of the ³⁵S labeled in vitro transcription/translation products of HIV-1 PR-RT sequence from three different patients.

Figure 4. Autoradiograph of the ³⁵S labeled in vitro transcription/translation products of HIV-1 PR-RT sequences amplified from a patient sample (Pat) and in vitro cultured HIV-1 virus (HXB2) in the presence or absence of protease inhibitors.

Figure 5. Autoradiograph of the ³⁵S labeled in vitro transcription/translation products of HIV-1 PR-RT sequences amplified from a patient samples (Pat 1, 2 and 3). The in vitro translation is performed in the presence of increasing concentrations of Ritonovir, a protease inhibitor.

Figure 6. Western blot of the thrombin substrate described in example 6 after processing of the substrate by thrombin in presence or absence of thrombin inhibitor.

Figure 7. Fluorescence of the thrombin substrate described in example 6 after processing of the substrate by thrombin in presence or absence of thrombin inhibitor and bound-free separation on a column.

## Claims

1. A method for determining resistance of an enzyme against an inhibiting activity of a compound comprising amplification of a nucleic acid encoding said enzyme or at least one functional fragment thereof further comprising *in vitro* translation of amplified nucleic acid, contacting translated enzyme or fragment with a substrate and said compound and measuring the inhibiting activity of said compound by measuring enzymatic activity.

2. A method for determining an inhibiting activity of a compound towards an enzyme comprising amplification of a nucleic acid encoding said enzyme or at least one functional fragment thereof further comprising *in vitro* translation of amplified nucleic acid, contacting translated enzyme or fragment with a substrate and said compound and measuring the inhibiting activity of said compound by measuring enzymatic activity.

3. A method according to claim 1 or 2 wherein said amplification comprises *in vitro* transcription.

4. A method according to anyone of claims 1 to 3 wherein said enzyme is derived from a virus.

5. A method according to claim 4 wherein said enzyme is derived from a retrovirus.

6. A method according to anyone of claims 1 to 5 wherein said enzyme comprises a protease.

7. A method according to claim 6 wherein said enzyme comprises a retroviral protease.

8. Use of a method according to anyone of claims 1 to 7 in screening test enzymes for their likelihood of being inhibited by a compound.

9. Use according to claim 8 for identifying an enzyme inhibitable by a compound.

10. An enzyme identifiable by a use according to claim 9.

11. Use of a method according to anyone of claims 1 to 7 in screening test compounds for their likelihood of having inhibiting activity towards an enzyme.

12. Use according to claim 11 for identifying a compound having inhibiting activity towards an enzyme.

13. A compound identifiable by a use according to claim 12.

14. Use of a compound according to claim 13 in preparing a pharmaceutical composition.

15. A pharmaceutical composition comprising a compound according to claim 13.
